# EUROPEAN PATENT SPECIFICATION

(11) **EP 1 100 432 B1**
(45) Date of publication and mention of the grant of the patent: **19.12.2007**
(21) Application number: 99937718.7
(22) Date of filing: 30.07.1999
(51) Int. Cl.: A61K 8/19, A61K 8/64, A61Q 1/06, A61Q 1/14, A61Q 3/02, A61Q 17/04, A61Q 19/10

(54) **NOVEL USES OF ASCORBYL-PHOSPHORYL-CHOLESTEROL AND COMPOSITIONS FOR PRACTICING SAME**
NEUE ANWENDUNGSGEBIETE VON ASCORBYL-PHOSPHORYL-CHOLESTEROL UND ZUBEREITUNGEN, UM DIESE UMZUSETZEN
NOUVELLES UTILISATIONS DE L'ASCORBYL-PHOSPHORYL-CHOLESTEROL ET COMPOSITIONS PERMETTANT DE METTRE EN OEUVRE CES NOUVELLES UTILISATIONS

(30) Priority: 30.07.1998 US 126191; 09.11.1998 US 189368
(43) Date of publication of application: 23.05.2001
(73) Proprietor: AVON PRODUCTS, INC., New York, NY 10020-1196 (US)
(72) Inventor: PTCHELINTSEV, Dmitri, Mahwah, NJ 07430 (US); DUFFY, John, A., West Milford, NJ 07480 (US); KALAFSKY, Robert, Ogdensburg, NJ 07439 (US); PAHLCK, Harold, E., Waldwick, NJ 07463 (US)
(74) Representative: Dr. Weitzel & Partner
(86) International application number: PCT/US1999/017420
(87) International publication number: WO 2000/006091

(56) References cited:
- WO-A-97/42960
- WO-A-98/50004
- US-A- 5 607 968
- US-A- 5 683 704
- US-A- 5 703 122

## Description

### BACKGROUND OF THE INVENTION

### 1. Field of the Invention

The present invention relates to novel topical compositions. More particularly, the present invention is directed to topical compositions that have 3'-(L-ascorbyl-2-o-phosphoryl)-cholesterol, 3'-(L-ascorbyl-3-o-phosphoryl)-cholesterol, and their derivatives (hereinafter collectively referred "APC compound" or "APC compounds") as well as an iron oxide component. The present invention also relates to a method of improving the appearance of skin.

### 2. Description of Related Art

U.S. Patent No. 4,939,128 to Kato et al. is directed to the use of phosphoric acid esters of ascorbic acid for the treatment of systemic diseases, and not for cosmetics, topical dermatological or skin uses. This patent provides that certain phosphoric acid esters of ascorbic acid display improved oxygen-scavenging properties. However, the specific mention of a cholestenyl group suggests that conjugates of L-ascorbic acid and cholesterol were neither practical nor desired. The Kato et al. patents fail to disclose phosphoric acid diesters of ascorbic acid and cholesterol, and the synergistic results that may be obtained in topical compositions through the incorporation of effective amounts of APC compounds.

U.S. Patent No. 5,607,968 to Ptchelintsev provides a method of making ascorbic acid-phosphoryl derivatives that incorporate straight chain (C₂ to C₁₈) alkyl groups.

Heretofore, consumers desired cosmetic compositions that have been synergistically improved by incorporating APC compounds therein. Consumers also desired additional methods of improving the health and appearance of their skin, nails and lips using such APC compounds.

### BRIEF SUMMARY OF THE INVENTION

The present invention relates to novel topical compositions. More particularly, the present invention is directed to topical compositions that have 3'-(L-ascorbyl-2-o-phosphoryl)-cholesterol, 3'-(L-ascorbyl-3-o-phosphoryl)-cholesterol, and their derivatives (hereinafter collectively referred "APC compound" or "APC compounds") as well as an iron oxide component. The present invention also relates to novel uses of topical compositions having APC compounds to enhance the appearance and health of skin, lips and nails. The present invention further relates to novel methods of topically administering compositions having APC compounds, such as with a lipstick or a dermal patch.

The present invention, in brief summary, is topical compositions having APC compounds, and methods of administering these topical compositions to improve the condition of skin, as well as its appearance.

### BRIEF DESCRIPTION OF THE DRAWING

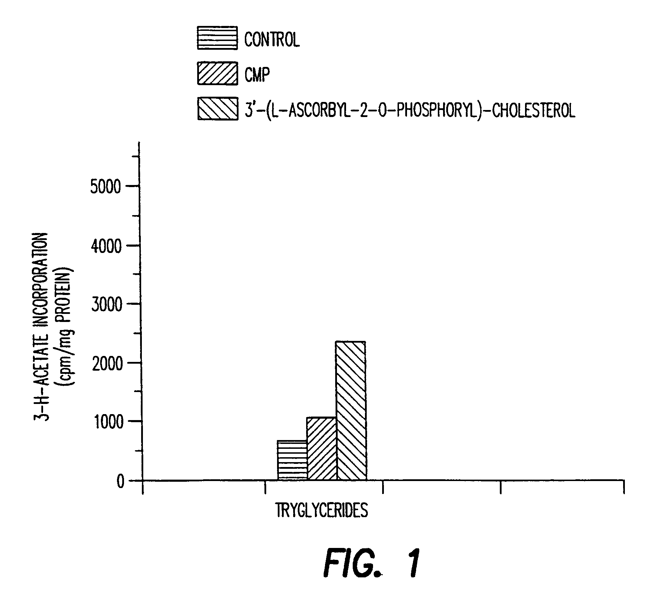

Fig. 1 illustrates that 3'-(L-ascorbyl-2-o-phosphoryl)-cholesterol significantly increases triglyceride synthesis in cultured human keratinocytes.

### DETAILED DESCRIPTION OF THE INVENTION

Although the individual benefits of ascorbic acid and cholesterol are known, mechanical mixing of L-ascorbic acid and cholesterol results in an unstable product due to the instability of L-ascorbic acid. Free L-ascorbic acid in topical preparations demonstrates poor stability and tends to break down due to oxidative and/or non-oxidative degradation. The degraded ascorbic acid loses activity and the resultant product loses aesthetic appeal since it exhibits a cosmetically undesired brown color.

However, APC compounds provide advantages not available from simple admixtures of ascorbic acid and cholesterol without the disadvantages associated with ascorbic acid. In the APC compounds, the conjugated ascorbic acid becomes resistant to degradation. This benefits the consumer by providing a more stable, more effective, and more aesthetically pleasing product.

In addition to the increased stability demonstrated by topical compositions having APC compounds rather than ascorbic acid, APC compounds also provide other advantages. For example, APC compounds are better absorbed through the skin since the cholesteryl group serves as a carrier moiety and facilitates delivery of polar ascorbic acid through the non-polar outermost protective layer of skin (i.e., the stratum corneum) and increases the bioavailability of the ascorbic acid in the topical composition.

Although the L-ascorbic acid is covalently bound to the cholesterol by a phosphoryl or phosphate group, natural enzymes, such as phosphatases that are present in the skin, gradually cleave the phosphoryl or phosphate linkage between cholesterol and ascorbic acid, resulting in sustained release of free L-ascorbic acid and cholesterol into the stratum corneum. The released cholesterol is a natural substrate for skin and supplements that otherwise produced by the body. Topically applied cholesterol improves elasticity, tone and resistance to drying.

U.S. Patent Nos. 5,866,147 and 5,922,335 as well as published PCT application WO 98/50004 provide stable topical compositions comprising the APC compounds, namely 3'-(L-ascorbyl-2-o-phosphoryl)-cholesterol, 3'-(L-ascorbyl-3-o-phosphoryl)-cholesterol, and their derivatives. APC compounds in topical compositions provide beneficial effects to the skin by decreasing abnormal elastin production, decreasing free-radical formation, and increasing keratinocyte triglyceride synthesis. APC compounds provide beneficial effects of both L-ascorbic acid and cholesterol on skin by providing increased collagen production and skin-lightening associated with ascorbic acid and by improving elasticity, resistance, tone and moisture retention of the skin associated with cholesterol.

APC compounds include 3'-(L-ascorbyl-2-o-phosphoryl)-cholesterol, 3'-(L-ascorbyl-3-o-phosphoryl)-cholesterol, functional or structural isomers thereof and salts thereof ("APC compounds"). The exemplary compounds include functional or structural isomers of 3'-(L-ascorbyl-2-o-phosphoryl)-cholesterol (Formula I) such as 3'-(L-ascorbyl-3-o-phosphoryl)-cholesterol (Formula II). Both formulas are illustrated below. 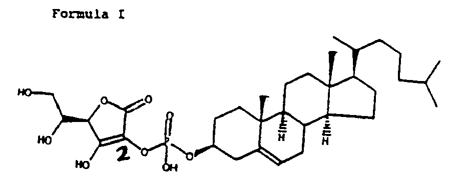 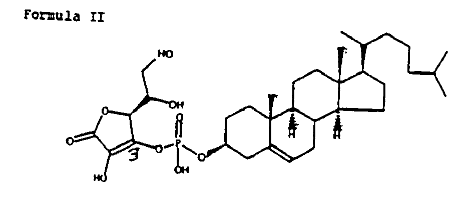 The preferred APC compound is 3'-(L-ascorbyl-2-o-phosphoryl)-cholesterol, salts thereof and derivatives thereof.

Exemplary salts of the APC compound include ammonium, calcium, lithium, potassium or sodium salts. Salts of the APC compounds can be incorporated, either individually or with the L-ascorbic derivative, into a topical vehicle. A salt with an organic amine, such as ethanolamine, may also be used in combination with the APC compound.

Although some benefits of APC compounds have been disclosed, the synergistic combinations of APC compounds with known cosmetic ingredients disclosed herein are novel.

The presence of both a hydrophilic portion (the ascorbyl group) and a lipophilic portion (the cholesteryl group) is believed to provide surfactant activity that aids in removal of cosmetics, dirt and oil from the skin, hair and nails. In addition, since APC compounds are useful in topical preparations or compositions for improving signs of dermatological aging, such as wrinkling in the eye areas or "crows feet," or fine wrinkles around the mouth area, the use of APC compounds in cosmetic cleansing or make-up remover compositions, especially on the sensitive skin areas around the eyes and mouth, will provide consumers with a single product having the dual benefits of cleansing and improving dermatological health. The cosmetic cleansing compositions may be in the form of a cream, a lotion, an emulsion, a gel, an ointment, a foam, a spray or a mousse. However, it is preferred that the cosmetic cleansing composition is a cream, a lotion or an emulsion.

A topical composition having APC compounds may be a moisturizer to alleviate dry skin. Although applicant does not wish to be bound to any theory, it is believed that APC compounds provide moisturizing benefits in at least one of two ways. When the cholesteryl group is cleaved from the APC compound, the cholesterol moiety is a natural substrate for the skin and is associated with increased moisture retention and increased suppleness exhibited by skin. Also, APC compounds have been found to increase keratinocyte triglyceride synthesis. Increased triglyceride synthesis is believed to enhance moisture retention and suppleness exhibited by skin. Most likely, both of the aforementioned factors contribute to the moisturizing effects.

Moisturizing topical compositions of the present invention have from about 0.1 wt% to about 20 wt% of the APC compound. More preferably, moisturizing topical compositions of the present invention have from about 1 wt% to about 10 wt% of the APC compound. U.S. Patent No. 5,866,147 and PCT application WO 98/5004 provide examples of lotions, emulsions, and microemulsions useful for practicing the method of moisturizing skin by topically applying compositions having APC compounds.

A preferred example of a topical composition of the present invention for use as a moisturizer comprises from about 2 wt% to about 5 wt%, more preferably about 2.5 wt%, of the APC compound in a suitable topical vehicle. If the moisturizer is to be applied for wear during daytime hours, the moisturizer preferably has from about 0.1 wt% to about 25 wt% of a sunscreen. In addition, the vehicle may contain additional conventional additives and adjuvents, which are discussed below.

As has been disclosed, topical application of APC compounds improves collagen synthesis. The topical application of APC compounds directly to the lip is expected to increase collagen synthesis and, thus, Improve the appearance of the lips. The increase in collagen synthesis is also expected to create fuller lips. Although APC compounds may be, or may be incorporated into, lip cosmetics, such as lipsticks, lip balms and pomades, it is preferred that the APC compound is, or is incorporated into, a lipstick composition. Lipsticks provide consumers with easy application precisely on the lip area. Lip cosmetics of the present invention are pigmented. However, as will be discussed below, the use of APC compounds in pigmented cosmetics, such as lip cosmetics, provides a novel method of improving the appearance and performance of pigmented cosmetics.

Cosmetic compositions often have pigments incorporated therein. An example of such pigments are iron oxides. Previously, the incorporation of ascorbic acid into a topical compositions having iron oxides usually resulted in an increase in oxidation. (The term "iron oxides" as used herein describes one or more iron oxides.) The increased oxidation, in turn, results in the cosmetic composition becoming discolored, less efficacious and, accordingly, less attractive to consumers. It has been found that when an APC compound is incorporated into cosmetic compositions containing iron oxides, there is unexpectedly little or no increase in the oxidation topically associated with ascorbic acid. Thus, incorporating the APC compound into an inventive cosmetic composition having iron oxides according to the independent claims is a novel method for improving the appearance of the skin while providing the advantages of both ascorbic acid and iron oxides.

An example of such an inventive topical composition may have from about 0.2 wt% to about 20 wt% of iron oxides in addition to the APC compound. One preferred example of such a topical composition has from about 5 wt% to about 7 wt% of iron oxides and about 1 wt% of the APC compound in a suitable vehicle. In the preferred example, the iron oxides are selected from the group consisting of iron oxide red 2259-preserved, iron oxides (yellow), iron oxides (black), and mixtures thereof.

The addition of one or more APC compounds to compositions enhances surface dispersion of pigments. As was discussed above, APC compounds have both hydrophilic and lipophilic groups. As a result APC compounds assist in decreasing surface tension that allows pigments to "wet out" or disperse effectively. When used with a pigment component, the APC compounds act as a pigment wetter, binder and disperser of the pigment.

It has been discovered that not only do APC compounds enhance the spreadability of topical pigmented compositions, but also that the topical pigmented compositions of the present invention exhibit a self-leveling effect. This self-leveling effect results in greater uniformity with regard to the thickness of the layer of the applied pigmented topical composition. While this self-leveling effect is beneficial to all pigmented compositions, it is particularly useful in topical pigmented compositions such as lipsticks, foundations, and nail enamels/polishes. Thus, topical compositions having APC compounds as a pigment additive are expected to exhibit superior coverage upon application. In addition, when the topical pigmented composition of the present invention is a cosmetic such as foundation, the addition of the APC compounds is expected to provide a superior feel upon application to the skin.

This dispersion assisting property of APC compounds is also believed to improve the coating characteristics of nail compositions. In addition, the use of APC compounds as, or in, nail coating compositions, such as nail enamels or polishes, is expected to enhance the gloss/shine exhibited once the nail coating composition has cured, and to also increase resistance to wear due to chipping, peeling and abrasion. The benefits of improving the health and appearance of the nails is not limited to the incorporation of APC compounds into nail coating compositions, such as nail enamels discussed above. In fact, it is believed that the APC compounds can themselves be the nail coating composition. In above-mentioned PCT WO 98/50004, it was disclosed that APC compounds are useful for treating disorders associated with the nails and cuticles. The overall nail health is improved by the topical application of the APC compounds. Thus, APC compounds into may be incorporated into nail health enhancing compositions, such as moisturizers, and even into nail polish removers.

In a nail composition, the APC compound is compatible with ingredients such as film-formers, sunscreens, plasticizers, solvents, suspending agents, pigments, and preservatives. A non-exclusive list of film formers includes nitrocellulose, cellulose acetate butyrate, polyurethanes and mixtures of polyurethanes with cellulose acetate butyrate or with nitrocellulose, acrylics, acrylates, polyurethanes, vinyl acrylates, and polyesters.

APC compounds are also compatible with both aqueous and nonaqueous solvents that are used in nail coating compositions. Examples of solvents include methanol, ethanol, propanol, butanol, carbonyl containing solvents such as acetone and butenone, acetates such as ethyl acetate, and chlorinated hydrocarbons such as methylene chloride.

The nail compositions of the present invention may include a further pigment component ("nail pigment component" beside iron oxid. Non-limiting examples of suitable organic or inorganic pigment components include carmine, bismuth oxychloride, zinc oxide, kaolin, ultramarine violet, ultramarine blue, talc, mica, titanium dioxide, any of the foregoing carried on the surface of talc, mica or titanium dioxide, or mixtures thereof.

PCT WO 98/50004 and U.S. Patent No. 5,922,335 disclose that APC compounds are compatible with sunscreens. It is believed that topical sunscreen compositions having both an APC compound and a sunscreen provide synergistic results. The sunscreen may be benzophenone-3 and derivatives thereof, butyl methoxydibenzoylmethane (a non-limiting example of the foregoing is available from Roche Inc. under the tradename "parasol-1789") and derivatives thereof, terephthalylidene dicamphor sulfonic acids (a non-limiting example of foregoing is available from L'Oreal under the tradename "MEXORYL SX") and derivatives thereof, titanium dioxide, ethyl methyl cinnamate, ethylhexyl methoxycinnamate, octocrylene and combinations thereof. The derivatives of the foregoing sunscreens may be their inorganic or organic salts.

The combination of sunscreens with APC compounds is expected to be particularly beneficial because APC compounds have been found to inhibit increased production of abnormal elastin (also known as "elastosis"). Since UV radiation is one cause of elastosis, the combination of the APC compound and the sunscreen will provide a superior topical sunscreen composition from the resulting synergism.

Preferably, the topical composition having a sunscreen, the APC compound and the iron oxid component will have from about 0.1 wt% to about 20 wt% of at least one APC compound and from about 0.1 wt% to about 25 wt% of at least one sunscreen. The sunscreen is preferably one of the above-disclosed sunscreens. The sunscreen is more preferably a mixture of two or more sunscreens, and most preferably provides both UVA and UVB protection.

The cosmetic cleansing compositions, the moisturizing compositions and the sunscreen compositions of the present invention may additionally have cosmetic additives and adjuvants selected especially from among fats, organic solvents, ionic or nonionic thickening agents, softeners, antioxidants and especially anti-free-radical antioxidants, opacifying agents, stabilizing agents, emollients, silicones, alpha-hydroxy acids, antifoaming agents, hydrating agents, vitamins, fragrances, preservatives, surfactants, fillers, insect repellants, sequestering agents, polymers, propellents, alkylinating or acidifying agents, dyes and colorants, or any other ingredients usually employed in the cosmetics embodiments disclosed above.

The APC compounds may be combined with sulfites. It has been found that the combination of APC compounds and sulfites in a topical composition uniquely maintains the color of the composition.

The APC compounds may be used as an anti-bacterial system, and as or in a hair conditioner, and as or in a hair shampoo, and as or in an anti-dandruff agent or composition.

One example of a composition comprising APC is set forth below as Background Example 1.

### Background EXAMPLE 1

| Ingredient | Wt% of the Total Composition | | |
|---|---|---|---|
| Sodium Ascorbyl/Cholesterol Phosphate | 0.75 | - | 6 |
| 3,6,9-Trioxaundecanedioic Acid | 2 | - | 12 |
| Ethylhexyl-methoxycinnamate | 2 | - | 7.5 |
| Benzophenone-3 | 2 | - | 6 |
| Butyl Methoxydibenzoylmethane | 2 | - | 3 |
| Tetrasodium EDTA | 0.05 | - | 0.25 |
| Glycerin | 2 | - | 5 |
| Hydroxyethyl Cellulose | 0.1 | - | 1.0 |
| Ammonium Hydroxide 30% | 0.5 | - | 4.0 |
| Sorbitan Tristearate | 0.5 | - | 3.0 |
| Cetearyl Glucoside | 0.5 | - | 4.0 |
| POE (100M) 6000 Monostearate | 0.5 | - | 4.0 |
| Methylparaben | 0.1 | - | 0.4 |
| Sodium Sulfite | 0.05 | - | 0.5 |
| Benzyl Alcohol | 0.1 | - | 1.0 |
| Cylclomethicone | 1 | - | 10 |
| Demineralized Water | qs | | |

### BACKGROUND EXAMPLE 2

This example summarizes a study which demonstrates the ability of ascorbyl-2-o-phosphoryl)-cholesterol to inhibit dermal elastin production as measured by elastin gene promotor activity. An art recognized method for evaluating the molecular events associated with increased elastin production was performed by measuring the activity of the elastin gene promoter, which is responsible for the activation of elastin gene transcription. (Bernstein, E. et al. 1995 Jour. Invest. Dermatol. 105:269-273).

Human elastin promoter molecules were linked to reporter molecules. The promoter-reporter linked molecules were then transfected into cultured dermal fibroblasts. Previous studies indicate that exposure of cultured fibroblasts to UVB radiation results in upregulation of elastin promoter activity. However, it is believed that UVA radiation may have an indirect effect on solar-induced elastosis. Hence, the degree of elastin promoter activity may be considered as an indicator of elastin production.

Solutions of 1µM to 1000µM of each 3'-(L-ascorbyl-2-o-phosphoryl)-cholesterol and ascorbic acid were produced. These solutions were then supplied to cultured fibroblasts. The control consisted of cultured fibroblasts in media containing neither 3'-(L-ascorbyl-2-o-phosphoryl)-cholesterol nor ascorbic acid.

The cultured fibroblasts were irradiated with single, dose of UVB radiation (5.5mJ/cm²⁾ using an FS-40 light source. The spectrum of effectiveness of the FS-40 light source is estimated at 80 to 90% attributable to UVB. It is believed that 10 to 20% of the effectiveness of the FS-40 light source is attributable to UVC. The activity attributable to UVA is believed to be negligible.

It was discovered that 3'-(L-ascorbyl-2-o-phosphoryl)-cholesterol decreased elastin promoter activity in cultured fibroblasts in a dose dependent manner. The effect of ascorbic acid on elastin promoter activity when compared to the results yielded by 3'-(L-ascorbyl-2-o-phosphoryl)-cholesterol demonstrate that 3'-(L-ascorbyl-2-o-phosphoryl)-cholesterol exhibits a far superior degree of elastin promoter inhibition and, thus, elastin formation.

In addition, the test results demonstrate that 3'-(L-ascorbyl-2-o-phosphoryl)-cholesterol inhibits elastin promoter activity for a significantly longer period of time than ascorbic acid. Table A below illustrates the relative activity of ascorbic acid and 3'-(L-ascorbyl-2-o-phosphoryl)-cholesterol.

**TABLE A**

| Comparison table of percent inhibition of elastin promoter activity of 3'-(L-ascorbyl-2-o-phosphoryl)-cholesterol and ascorbic acid at 6 hour and 12 hour intervals. | | |
|---|---|---|
| 100µM Solution of | % Inhibition at 6 hours | % Inhibition at 12 hours |
| 3'-(L-ascorbyl-2-o-phosphoryl)-cholesterol | 43 | 34 |
| Ascorbic Acid | 23 | 0 |

### BACKGROUND EXAMPLE 3

Normal human keratinocytes (hereinafter "NHK") were cultured and then divided into three groups. Each of the three groups were grown in various media for 18.5 hours, the various media being as follows:

| Group I: | KGM |
|---|---|
| | 0.07mM calcium |
| | 0.05 wt% ethanol |
| | 0.25 wt% methanol |
| | 1 µg/ml Vitamin E |
| | 1 mM "cold" sodium acetate |
| | |

| Group II: | KGM |
|---|---|
| | 0.07mM calcium |
| | 0.05 wt% ethanol |
| | 0.25 wt% methanol |
| | 1 µg/ml Vitamin E |
| | 1mM "cold" sodium acetate |
| | 10mM Cholesterylmonophosphate (CMP) |
| | |

| Group III: | KGM |
|---|---|
| | 0.07mM calcium |
| | 0.05 wt% ethanol |
| | 0.25 wt% methanol |
| | 1 µg/ml Vitamin E |
| | 1mM "cold" sodium acetate |
| | 10mM 3'-(L-ascorbyl-2-o-phosphoryl)-cholesterol |

At the conclusion of 18.5 hours, 3H-acetate sodium salt (product number NEN#003H, 6.08 ci/mMole) was added to each group at 3uCi/ml. The three groups were incubated at 37°C for an additional 3 hours.

At the conclusion of the incubation period, each group was washed three times with iced PBS. The cells were then collected, sonicated and aliquated for lipid extraction using Bligh/Dyer method and for protein quantification. The lipid extracts were subfracationated by Thin Layer Chromatography. Proteins were quantitated by Bradford assay. Measurement of triglyceride synthesis is expressed as counts per minute per milligram of 3-H acetate.

Triglyceride biosythesis in cultured human keratinocytes is measured as a function of incorporation of 3-H acetate. The incorporation of 3-H acetate is expressed as counts per minute per milligram of 3-H acetate (cpm/mg protein). The results are illustrated in Fig. 1. As demonstrated in Fig. 1, 3'-(L-ascorbyl-2-o-phosphoryl)-cholesterol significantly increases triglyceride synthesis in cultured keratinocytes.

### BACKGROUND EXAMPLE 4

Hydroxy radical scavenging activity was measured according to an art accepted method. (See Liu et al. Antioxidant Action of Guilingji in the Brain of Rats with FeCl3-induced Epilepsy, Free Radical Biol. & Med., (9:451-545, 1990.) Sample solutions of 3'-(L-ascorbyl-2-o-phosphoryl)-cholesterol of varying concentrations were prepared by dissolving 3'-(L-ascorbyl-2-o-phosphoryl)-cholesterol in 0.1 M potassium phosphate buffer (pH=7.4). The following solutions were also prepared: 0.18M 5,5-dimethyl-1-pyrooline-N-oxide (hereinafter "DMPO") in 0.1 M potassium phosphate buffer; 2mM hydrogen peroxide (hereinafter "H₂O₂") in 0.1 M potassium phosphate buffer; and 0.2mM iron(II) sulfate (hereinafter "FeSO₄") in distilled water. Fifty (50) ml of each of the aforedescribed solutions and 50 ml of a sample solution were mixed for thirty (30) seconds and then placed in a flat cell. The Electron Spin Resonance was then measured using Free Radical Monitor JES-FR30, manufactured by JEOL, Tokyo, Japan, (hereinafter "FRM"). The FRM normalized all spectra for calculation by using an internal standard of manganese dioxide (hereinafter "MnO₂"). All peak heights were compared to the constant MnO₂ signal generated. Each sample peak height was then divided by the MnO₂ peak height to provide a relative peak height (hereinafter "RPH"). The results are set forth in Table B.

**TABLE B**

| Hydroxyl radical scavenging activity of 3'-(L-ascorbyl-2-o-phosphoryl)-cholesterol as measured by Relative Peak Height (RPH) of sample as compared with MnO₂. | | |
|---|---|---|
| Concentration of 3'-(L-ascorbyl-2-o-phosphoryl) -cholesterol (mg/ml) | Peak Height | Relative Peak Height (RPH) |
| 2.5000 | 0.09 | 5.6 |
| 1.2500 | 0.15 | 9.4 |
| 0.6250 | 0.49 | 30.6 |
| 0.3125 | 0.92 | 57.5 |
| 0.1563 | 1.25 | 78.1 |
| 0.0782 | 1.36 | 85.0 |
| 0.0391 | 1.60 | 100.0 |
| 0.0000 (Control) | 1.60 | 100.0 |

As demonstrated by the results set forth in Table B, 3'-(L-ascorbyl-2-o-phosphoryl)-cholesterol is an effective scavenger of hydroxyl radicals.

### BACKGROUND EXAMPLE 5

Superoxide anion scavenging ability was estimated according to the method described in the Liu et al. article noted above. Superoxide anion was generated by a hypoxanthine (hereinafter "HPX")-xanthine oxidase (hereinafter "XOD") system. The following solutions were prepared: 50 µl of 4 mM HPX in 0.1M potassium phosphate buffer; 30µl of dimethyl sulfoxide, 50µl of sample; 20µl OF 4.5mL DMPO in 0.1M potassium phosphate buffer; and, 50µl of XOD (0.4units/ml) in 0.1 M potassium phosphate buffer. The pH of the 0.1 M potassium phosphate buffer was 7.4. The solutions of HPX, DMSO, DMPO, XOD and sample were mixed and transferred into a 200µl-capacity flat cell. Electron Spin Resonance spectra was analyzed. It was demonstrated that at the concentration of about 2.5mg/ml, 3'-(L-ascorbyl-2-o-phosphoryl)-cholesterol inhibited the formation of the superoxide by 50%.

## Claims

1. A method of improving the appearance of skin comprising the step of applying to the skin a composition comprising:
an iron oxide component; and
an effective amount of a compound selected from the group consisting of 3'-(L-ascorbyl-2-o-phosphoryl)-cholesterol, 3'-(L-ascorbyl-3-o-phosphoryl)-cholesterol, isomers thereof, salts thereof and mixtures thereof.

2. A composition having an iron oxide component and comprising an effective amount of a compound selected from the group consisting of 3'-(L-ascorbyl-2-o-phosphoryl)-cholesterol, 3'-(L-ascorbyl-3-o-phosphoryl)-cholesterol, isomers thereof, salts thereof and mixtures thereof in a suitable vehicle, wherein the composition is selected from the group consisting of a lip cosmetic, a nail coating composition, a nail enamel, a make-up remover and a photoprotective topical composition.

3. The composition of claim 2, wherein the lip cosmetic is a lipstick.

4. The composition of claim 2, wherein the photoprotective topical composition comprises a sunscreen selected from the group consisting of benzophenone-3 and derivatives thereof, butyl methoxydibenzoylmethane and derivatives thereof, terephthalylidene dicamphor sulfonic acids and derivatives thereof, titanium dioxide, ethyl methyl cinnamate, ethylhexyl-methoxycinnamate, octocrylene, and mixtures thereof.

## Patentansprüche

1. Verfahren zur Verbesserung des Aussehens der Haut, umfassend den Schritt Auftragen einer Zusammensetzung, umfassend:
eine Eisenoxid-Komponente; und
eine wirksame Menge einer Verbindung, ausgewählt aus der Gruppe, bestehend aus 3'-(L-Ascorbyl-2-o-phosphoryl)-cholesterin, 3'-(L-Ascorbyl-3-o-phosphoryl)-cholesterin, deren Isomeren, Salzen und Gemischen, auf die Haut.

2. Zusammensetzung mit einer Eisenoxidkomponente und mit einer wirksamen Menge einer Verbindung, ausgewählt aus der Gruppe, bestehend aus 3'-(L-Ascorbyl-2-o-phosphoryl)-cholesterin, 3'-(L-Ascorbyl-3-o-phosphoryl)-cholesterin, deren Isomeren, Salzen und Gemischen, in einem geeigneten Vehikel, wobei die Zusammensetzung ausgewählt ist aus der Gruppe, bestehend aus einer Lippenkosmetik, Nagelüberzug-Zusammensetzung, einem Nagellack, Make-up-Entferner und einer topischen Lichtschutz-Zusammensetzung.

3. Zusammensetzung nach Anspruch 2, wobei die Lippenkosmetik ein Lippenstift ist.

4. Zusammensetzung nach Anspruch 2,wobei die topische Lichtschutz-Zusammensetzung ein Sonnenschutzmittel umfasst, ausgewählt aus der Gruppe, bestehend aus Benzophenon-3 und Derivaten davon, Butylmethoxydibenzoylmethan und Derivaten davon, Terephthalidendikampfersulfonsäuren und Derivaten davon, Titandioxid, Ethylmethylcinnamat, Ethylhexylmethoxycinnamat, Octocrylen und Gemischen davon.

## Revendications

1. Procédé d'amélioration de l'apparence de la peau comprenant l'étape consistant à appliquer à la peau une composition comprenant :
un composant d'oxyde de fer ; et
une quantité efficace d'un composé choisi parmi le groupe constitué du 3'-(L-ascorbyl-2-o-phosphoryl)-cholestérol, du 3'-(L-ascorbyl-3-o-phosphoryl)-cholestérol, d'isomères de ceux-ci, de sels de ceux-ci et de mélanges de ceux-ci.

2. Composition comprenant un composant d'oxyde de fer et une quantité efficace d'un composé choisi parmi le groupe constitué du 3'-(L-ascorbyl-2-o-phosphoryl)-cholestérol, du 3'-(L-ascorbyl-3-o-phosphoryl)-cholestérol, d'isomères de ceux-ci, de sels de ceux-ci et de mélanges de ceux-ci dans un excipient approprié, dans laquelle la composition est choisie parmi le groupe constitué d'un produit cosmétique pour les lèvres, d'une composition de revêtement pour les ongles, d'un vernis à ongles, d'un démaquillant et d'une composition topique photoprotectrice.

3. Composition selon la revendication 2, dans laquelle le produit cosmétique pour les lèvres est un rouge à lèvres.

4. Composition selon la revendication 2, dans laquelle la composition topique photoprotectrice comprend un écran solaire choisi parmi le groupe constitué de la benzophénone-3 et de dérivés de celle-ci, du butyl méthoxydibenzoylméthane et de dérivés de celui-ci, d'acides téréphtalylidène dicamphre sulfoniques et de dérivés de ceux-ci, du dioxyde de titane, du cinnamate d'éthylméthyle, de l'éthylhexylméthoxycinnamate, de l'octocrylène et de mélanges de ceux-ci.
